# EUROPÄISCHE PATENTSCHRIFT

(11) **EP 0 738 252 B1**
(45) Veröffentlichungstag und Bekanntmachung des Hinweises auf die Patenterteilung: **14.04.1999**
(21) Anmeldenummer: 95935940.7
(22) Anmeldetag: 16.10.1995
(51) Int. Cl.: C07C 31/18, C07C 31/26, A23G 3/30, A61K 9/20

(54) **DURCH CO-SPRÜHTROCKNUNG ERHÄLTLICHE POLYOL-ZUSAMMENSETZUNG**
POLYOL COMPOSITION PRODUCED BY CO-SPRAY-DRYING
COMPOSITION DE POLYOLS OBTENUE PAR CO-SECHAGE PAR PULVERISATION

(30) Priorität: 08.11.1994 DE 4439858
(43) Veröffentlichungstag der Anmeldung: 23.10.1996
(73) Patentinhaber: MERCK PATENT GmbH, 64293 Darmstadt (DE)
(72) Erfinder: SCHWARZ, Eugen, D-64625 Bensheim (DE); MÖSCHL, Gernot, D-64331 Weiterstadt (DE); NIKOLAUS, Heinrich, D-64291 Darmstadt (DE); STEINSTRÄSSER, Ralf, D-64380 Ro dorf (DE)
(86) Internationale Anmeldenummer: EP9504059
(87) Internationale Veröffentlichungsnummer: WO9614282

(56) Entgegenhaltungen:
- EP-A- 0 111 209
- EP-A- 0 380 219
- EP-A- 0 413 427
- EP-A- 0 497 439
- DD-A- 277 176
- DE-A- 2 809 536
- US-A- 5 158 789

## Beschreibung

Die Erfindung betrifft eine durch Co-Sprühtrocknung erhältliche Polyol-Zusammensetzung.

Polyole und Polyolmischungen werden in großem Umfang als Zusatzstoffe und Trägerstoffe unter anderem für Kau- und Lutschtabletten, Kaugummi und andere Produkte der Süßwarenindustrie verwendet. Der besondere Vorteil von Polyolen liegt darin, daß sie im Prinzip auch zum direkten Verpressen ohne weitere Hilfs- und Zusatzstoffe geeignet sind. Gewonnen werden Polyole in der Regel durch Hydrierung der ihnen zugrundeliegenden Kohlehydrate. In fester Form können sie sowohl durch Kristallisation als auch durch Sprühtrocknung erhalten werden.

Bei der Herstellung von Komprimaten ergeben solche Polyole in der Regel eine rauhe wenig zufriedenstellende Oberfläche bzw. im Falle von Polyolgemischen zudem eine geringe Härte. Es sind daher spezielle Verfahren entwickelt worden, um zur besseren Verpressung geeignete Polyole herzustellen.

In der DE 32 45 170 wird vorgeschlagen, eine Polyolkombination aus Sorbit und 10 bis 15 Gew.% Mannit durch Sprühtrocknung herzustellen. Dadurch soll die Biegefestigkeit von Tabletten erhöht werden. Es findet sich dort kein Hinweis, daß mit durch Sprühtrocknung erhältlichen Polyolkombinationen mit anderen Polyolen oder geringeren Mannitanteilen verbesserte Eigenschaften, insbesondere höhere Plastizität erzielt werden kann.

Desweiteren ist ein solches Polyol zur Herstellung von Kaugummi weniger geeignet, da diese nach kurzer Kauzeit zu hart werden.

In der EP 0 528 604 wird eine durch Co-Schmelzen erhältliche Zusammensetzung aus Sorbit und Xylit beschrieben. Diese führt jedoch zu Tabletten mit vergleichsweise geringer Härte.

Es bestand daher die Aufgabe, eine Polyol-Zusammensetzung zur Verfügung zu stellen, die problemlos herstellbar ist und deren Tablettiereigenschaften und Plastizität gegenüber bekannten Polyolen verbessert ist.

Es wurde nun gefunden, daß eine durch Co-Sprühtrocknung erhältliche Polyol-Zusammensetzung enthaltend weniger als 10 Gew.% Mannit beim Tablettieren bei gleichem Preßdruck eine viel glattere Oberfläche ergibt und daß sich dieses Produkt zu Kaugummis verarbeitet läßt, welche bessere Verarbeitungseigenschaften aufweisen und viel länger weich bleiben als mit herkömmlichem Sorbit oder Mischungen aus Sorbit und weiteren Polyolen hergestelltem Kaugummi.

Gegenstand der Erfindung ist somit eine im wesentlichen aus mindestens zwei Polyolen durch Co-Sprühtrocknung erhältliche Zusammensetzung, welche weniger als 10 Gew.% Mannit enthält.

Der Begriff Polyol steht für Zuckeralkohole der allgemeinen Formel

CH₂OH-(CHOH)ₙ-CH₂OH,

wobei n für 2 bis 6, vorzugsweise 3 bis 4, steht,
sowie deren dimeren Anhydride; insbesondere C₁₂H₂₄O₁₁.

Insbesondere steht der Begriff Polyole für Hexite wie Sorbit und Mannit, Pentite wie Xylit möglich sind aber auch C₄-Polyalkohole wie Erythit oder C₁₂-Polyalkohole wie Lactit. Der Begriff Polyol-Zusammensetzung steht für eine Zusammensetzung aus mehreren Polyolen die sich in ihrer Zusammensetzung von bei der technischen Herstellung von Sorbit anfallenden Zusammensetzungen deutlich unterscheiden, vorzugsweise solche Zusammensetzungen, die mindestens zwei Poyole mit unterschiedlicher Anzahl von C-Atomen enthalten, insbesondere steht der Begriff für eine Zusammensetzung enthaltend mindestens ein Hexit und mindestens ein Pentit.

Bevorzugte Ausführungsformen sind
a) Zusammensetzungen, erhältlich durch Lösen von mindestens zwei Polyolen in Wasser und Versprühen des erhaltenen wässerigen Gemisches in einem Luftstrom mit einer Temperatur von 120 bis 300 °C.
b) Zusammensetzungen, wobei Sorbit und Xylit oder Sorbit, Xylit und Mannit als Polyole eingesetzt werden.
c) Zusammensetzungen, wobei das Verhältnis von Sorbit zu Xylit in einem Bereich zwischen 50 : 50 bis 99 : 1, insbesondere zwischen 65 : 35 bis 98 : 2 liegt.
d) Zusammensetzungen, wobei das Verhältnis Sorbit : Xylit : Mannit in einem Bereich zwischen 90 : 1 : 9 bzw. 70 : 29 : 1 bis 98 : 1 : 1, insbesondere zwischen 90 : 2 : 8 bzw. 80 : 18 : 2 bis 94 : 1 : 5 bzw. 94 : 5 : 1, liegt.
e) Zusammensetzung, wobei der Gehalt an Wasser niedriger als 1 Gew.% liegt.
f) Zusammensetzung, welche bei Lagerung in einem Hygrostaten bei 55 % relativer Feuchte innerhalb von 24 Stunden mehr als 2 % Wasser aufnimmt.

Ein weiterer Gegenstand der Erfindung sind Komprimate, wie Lutsch- oder Kautabletten, sowie Kaugummis, sowie Süßwaren enthaltend eine erfindungsgemäße Zusammensetzung.

Weiterhin Gegenstand der Erfindung ist ein Verfahren zur Herstellung einer im wesentlichen aus mindestens zwei Polyolen bestehenden Zusammensetzung, beinhaltend die folgenden Schritte:
a) Herstellen einer wäßrigen Lösung von mindestens zwei Polyolen, wobei diese Lösung einen Mannitgehalt von weniger als 10 Gew.% bezogen auf den Gesamtpolyolgehat enthält,
b) Versprühen der erhaltenen Lösung in einem aufsteigendem Luftstrom mit einer Temperatur zwischen 120 und 300 °C, wobei das Wasser verdampft wird,
c) Isolierung der Zusammensetzung.

In einer besonders bevorzugten Ausführungsform besteht die erfindungsgemäße Polyol-Zusammensetzung im wesentlichen aus 85 bis 95 Gew.%, insbesondere 88 bis 94 Gew.%, Sorbit, und 5 bis 15 Gew.%, insbesondere 6 bis 12 Gew.%, aus einem oder zwei Polyolen ausgewählt aus Xylit und Mannit.

Vorzugsweise enthält die erfindungsgemäße Polyol-Zusammensetzung weniger als 10 Gew.%, insbesondere weniger als 5 Gew.% Mannit.

Zur Sprühtrocknung wird eine wäßrige Lösung von mindestens zwei Polyolen verwendet. Der Feststoffgehalt wird zuvor vorzugsweise durch Mischen zweier oder mehrerer Polyol-Lösungen im gewünschten Verhältnis auf etwa 30 bis etwa 75 Gew.%, insbesondere 60 bis 72 Gew.%, eingestellt. Die Versprühung wird durch Zerstäuben mittels Düsen, vorzugsweise mittels eines Zentrifugalzerstäubers in einen auf eine Temperatur von 120-300 °C, vorzugsweise 140-170 °C erwärmten trockenen zentrifugal eingeblasenen Luftstrom durchgeführt. Die Menge der zugeführten Polyollösung und der eingeblasenen Heißluft wird so abgestimmt, daß das Polyol bis auf einen Wassergehalt von etwa 0,3 bis etwa 1 Gew.% getrocknet wird. Auf jeden Fall sollte der Wassergehalt unterhalb 1 Gew.% liegen.

Die Polyolteilchen, die dabei durch Entwässerung der Polyollösungströpfchen erhalten werden, werden bei der Sprühtrocknung auf eine Temperatur von etwa 50 bis etwa 70 °C erwärmt, während sich die eingeblasene Luft auf etwa die gleiche Temperatur abkühlt. Die Polyolzusammensetzung wird in Behältern gesammelt und ist nach dem Abkühlen direkt zur Herstellung von Komprimaten oder Kaugummi geeignet.

Die erfindungsgemäße Polyol-Zusammensetzung weist ein homogenes Erscheinungsbild auf. Die Schüttdichte (nach DIN 53 912) beträgt etwa 0,3 bis 0,6 g/ml, die Stampfdichte (nach DIN 53 194) etwa 0,4 bis 0,7 g/ml. Die Teilchengröße kann durch das Sprühtrockenverfahren in weiten Grenzen gesteuert werden.

Die so charakterisierte Polyol-Zusammensetzung besitzt eine Reihe von vorteilhaften Tablettiereigenschaften:

Überraschenderweise kann festgestellt werden, daß mit der erfindungsgemäßen Polyol-Zusammensetzung bei gleicher Preßkraft härtere Tabletten mit deutlich glatterer Oberfläche hergestellt werden können, als mit den bekannten verpressbaren Sorbittypen bzw. durch mechanische Verreibung oder Co-Kristallisation erhältliche Polyolkombinationen. Da die optimale Festigkeit von Lutschtabletten durch das Lutschverhalten vorgegeben ist, bedeutet dies, daß optimal glatte, harte Tabletten bereits mit sehr niedrigen Preßkräften hergestellt werden können. Tablettiermaschinen, mit denen die erfindungsgemäße Polyol-Zusammensetzung verpreßt wird, können also bei relativ niedrigen Presskräften arbeiten und unterliegen auf diese Weise einem geringeren Verschleiß.

Durch die unregelmäßige Oberfläche ist die erfindungsgemäße Polyol-Zusammensetzung in der Lage, auch größere Mengen von Zusatzstoffen, wie z.B. von Kakaopulver, Farbstoffen oder anderen Zusätze zu binden. Auch bei starker Beladung mit Zusatzstoffen erhält man homogene Mischungen und die daraus hergestellten Komprimate besitzen ein gleichmäßiges Aussehen.

Aufgrund der besonderen Herstellungsart durch Versprühen einer wässerigen Lösung ist es möglich, wasserlösliche Zusätze, wie z.B. Zitronensäure, Süßstoffe, insbesondere Acesulfarm K, Aspartam, Saccharin, Cyclamat und Sucralose, Farbstoffe, Vitamine, insbesondere Ascorbinsäure und dergleichen, völlig homogen in der Polyol-Zusammensetzung bzw. den daraus hergestellten Komprimaten zu verteilen.

Neben der erfindungsgemäßen Polyol-Zusammensetzung enthalten die erfindungsgemäßen Komprimate einen oder mehrere Bestandteile ausgewählt aus:
pharmazeutischen Wirkstoffen und lebensmittelrechtlich zugelassenen Stoffen. Bevorzugte lebensmittelrechtliche zugelassene Stoffe sind natürliche, naturidentische oder künstliche Aroma- oder Geschmacksstoffe, Vitamine, Spurenelemente, Mineralien, Farbstoffe, Gleit-, Trennmittel, Süßstoffe, Stabilisatoren oder Antioxidantien. Der Anteil dieses Bestandteils liegt vorzugsweise zwischen 0,1 und 80 %, insbesondere zwischen 0,1 und 30 %.

Besonders bevorzugt sind Vitamintabletten mit einem oder mehreren Vitaminen.

Die Herstellung dieser Komprimate erfolgt an sich bekannter Weise durch Vermischen der Bestandteile in trockener Form und anschließender Tablettierung.

Die erfindungsgemäßen Polyol-Zusammensetzungen weisen eine wesentlich höhere Hygroskopizität als herkömmliche Polyole auf. Aufgrund dieser Eigenschaft eignen sie sich insbesondere für die Herstellung von Kaugummi.

Die erfindungsgemäßen Kaugummi enthalten neben der Polyolkombination eine lebensmittelrechtlich zugelassene Gum Base, einen oder mehrere flüssige Polyalkohole, insbesondere flüssiges Sorbit oder Glycerin und gegebenenfalls einen oder mehrere natürliche, naturidentische oder künstliche Aromastoffe.

In der Regel bestehen diese Kaugummi im wesentlichen aus:

| | |
|---|---|
| 15-35 Gew.% | Gum Base |
| 40-75 Gew.% | Sprühgetrocknete Polyol-Zusammensetzung |
| 15-25 Gew.% | eines oder mehrerer flüssiger Polyalkohole |
| 0- 5 Gew.%, | vorzugsweise 0,5 bis 2 Gew.% eines oder mehrerer Aromastoffe |

Die erfindungsgemäße Polyol-Zusammensetzung kann allein oder mit Zusätzen zu allen üblichen Zwecken eingesetzt werden, insbesondere zur Herstellung von Kau- und Lutschtabletten sowie von Kaugummi. Durch die verbesserten Tablettiereigenschaften wird durch die Erfindung ein erheblicher Fortschritt auf diesem Gebiet erzielt.

### Herstellungsbeispiele:

### Beispiel 1:

Eine 70%ige wäßrige Lösung, die bezogen auf die Trockenmasse 92,5 Teile Sorbit und 7,5 Teile Xylit enthält, wird hergestellt.

Diese Polyollösung wird bei etwa 40 °C mittels eines Zentrifugalzerstäubers in den oberen Teil eines zylindrisches Edelstahlturms gesprüht. Gleichzeitig wird auf 160 °C erhitzte Luft sowie kristallisiertes Polyol tangential in die Sprühzone eingeblasen. Dadurch trocknen die einzelnen Polyoltröpfchen aus und kristallisieren. Der Feststoffstrom wird über eine Kühltrommel abgeführt und dann geteilt: Ein Teil wird in die Sprühzone des Turmes zurückgeführt und der Rest gesiebt, über ein Fließbett nachgetrocknet und anschließend abgefüllt. Das so erhaltene Produkt läßt sich problemlos verpressen und führt zu Tabletten mit sehr glatter Oberfläche, sowie zu Kaugummi mit den erwähnten Vorteilen.

### Beispiel 2:

Eine 70%ige wäßrige Lösung, die bezogen auf die Trockenmasse 92 Teile Sorbit, 5 Teile Xylit und 3 Teile Mannit enthält, wird hergestellt. Das durch Sprühtrocknung analog Beispiel 1 erhaltene Produkt läßt sich problemlos verpressen, wobei Ergebnisse analog den in Beispiel 1 angegebenen erzielt werden.

In den folgenden Anwendungsbeispielen wird eine nach Beispiel 1 oder Beispiel 2 hergestellte Polyol-Zusammensetzung eingesetzt.

### Beispiel 3: Mentholtabletten

| | |
|---|---|
| Polyol-Zusammensetzung | 247,0 Gew.teile |
| Menthol | 1,8 Gew.teile |
| Magnesiumstearat | 1,2 Gew.teile |

Die Bestandteile werden vermischt und bei einem Preßdruck von 14 KN zu Tabletten von 9 mm Durchmesser und einem Gewicht von 250 mg verpreßt.

### Beispiel 4: Tabletten zur Kariesprophylaxe

| | |
|---|---|
| Cetylaminhydrofluorid | 41,82 Gew.teile |
| N-Cetylpyridiniumchlorid | 18,00 Gew.teile |
| Pfefferminzaroma | 40,00 Gew.teile |
| Polyol-Zusammensetzung | 1335,18 Gew.teile |
| Natriumhydrogencarbonat | 825,00 Gew.teile |
| Zitronensäure | 500,00 Gew.teile |
| Fumarsäure | 240,00 Gew.teile |

Die Bestandteile werden vermischt und bei einem Preßdruck von 25 KN zu Tabletten von 20 mm Durchmesser und 3000 mg Gewicht verpreßt.

### Beispiel 5: Lutschtabletten

| | |
|---|---|
| Polyol-Zusammensetzung hergestellt nach Beispiel 2 unter Zusatz von 0,8 Gew.% an Zitronensäure, bezogen auf eingesetztes Sorbit | 491,0 Gew.teile |
| Früchtetrockenaroma (verschiedene Geschmacksrichtungen) | 1,5 Gew.teile |
| Magnesiumstearat | 2,5 Gew.teile |

Die Bestandteile werden vermischt und bei einem Preßdruck von 30 KN zu Tabletten von 13 mm Durchmesser und 500 mg Gewicht verpreßt.

### Beispiel 6: Vitamin-C-Tabletten

| | |
|---|---|
| Ascorbinsäure | 105,0 Gew.teile |
| Orangenaroma | 10,0 Gew.teile |
| Polyol-Zusammensetzung | 1377,5 Gew.teile |
| hergestellt nach Beispiel 2 | |
| Magnesiumstearat | 7,5 Gew.teile |

Die Bestandteile werden vermischt und bei einem Preßdruck von 11 KN zu Tabletten von 18 mm Durchmesser und 1500 mg Gewicht verpreßt.

### Beispiel 7: Kaffeetabletten

| | |
|---|---|
| Polyol-Zusammensetzung | 462,5 Gew.teile |
| Kaffeeextraktpulver | 25,0 Gew.teile |
| Koffein | 10,0 Gew.teile |
| Magnesiumstearat | 2,5 Gew.teile |

Die Bestandteile werden vermischt und bei einem Preßdruck von 30 KN zu Tabletten von 13 mm Durchmesser und 500 mg Gewicht verpreßt.

### Beispiel 8: Multivitamin-Tabletten

| Vitaminmischung: |
|---|
| Thiaminnitrat |
| Riboflavin |
| Nicotinamid |
| Pyridoxolhydrochlorid |
| Vitamin B 12 (0,1 %) |
| Vitamin A (325.000 I.E./g) |
| Vitamin D 3 (100.000 I.E./g) |
| Vitamin C (überzogen) |
| Natriumascorbat |
| Vitamin E-acetat (50 %) |

| Tablettiermischung: | |
|---|---|
| Vitaminmischung | 147,40 Gew.teile |
| Polyol-Zusammensetzung erhältlich gemäß Beispiel 2 unter Zusatz von 0,3 Gew.% Aspartam, bezogen auf eingesetztes Sorbit | 563,29 Gew.teile |
| Erdbeeraroma | 2,00 Gew.teile |
| Farbstoff | 0,20 Gew.teile |
| Magnesiumstearat | 22,11 Gew.teile |

Die Bestandteile werden gemischt und bei einem Preßdruck von 11 KN zu Tabletten von 737 mg Gewicht verpreßt.

### Beispiel 9: Untersuchung der Tablettiereigenschaften

Es werden mit verschiedenen Polyolen Tabletten hergestellt:

| | | | |
|---|---|---|---|
| Tablettendurchmesser | 11 mm | Tablettengewicht | 450 mg |
| Tablettenhöhe | 3,7 bis 3,9 mm | Preßdruck | 12,5 KN |

| | |
|---|---|
| Polyol | 99,5 Gew.teile |
| Magnesiumstearat | 0,5 Gew.teile |

| | | | | |
|---|---|---|---|---|
| Polyol Co-Sprühung aus Beispiel 2 | Mech. Polyolmischung (mit einer Zusammensetzung wie Beispiel 2, jedoch aus sprühgetrocknetem Sorbit) | Mech. Polyolmischung (mit einer Zusammensetzung wie Beispiel 2 aus kristallisiertem Sorbit) | Reinsorbit sprühgetrocknet | Reinsorbit kristallisiert |

### Tablettenhärte:

| | | | | |
|---|---|---|---|---|
| 442 N | 231 N | 218 N | 280 N | 235 N |

### Lutschverhalten der Tabletten:

| | | | | |
|---|---|---|---|---|
| sehr glatt, geschmeidig | deutlich rauher | deutlich rauher | deutlich rauher | sehr deutl. rauher |

### Notwendiger Preßdruck um eine Tablettenhärte von etwa 150 N zu erreichen (der notwendige Preßdruck wird zum Beurteilungskritenium):

### Notwendiger Preßdruck:

| | | | | |
|---|---|---|---|---|
| 5100 N | 7400 N | 10200 N | 6800 N | 8400 N |

### Lutschverhalten der Tabletten:

| | | | | |
|---|---|---|---|---|
| glatt | merklich rauher | merklich rauher | merklich rauher | deutlich rauher |

### Beispiel 10: Spearmint-Kaugummi

| Rahmenrezeptur: | |
|---|---|
| Gum Base | 26,0 % |
| Polyol-Zusammensetzung | 52,5 % |
| Sorbitol F flüssig | 16,0 % |
| Glycerin | 4,0 % |
| Spearmint-Aroma | 1 ,5 % |

### Beispiel 10: Polyol-Zusammensetzung aus Beispiel 2

- Vergleichsbeispiel A:: Reines Sorbit anstelle der Polyol-Zusammensetzung
- Vergleichsbeispiel B:: Mechanische Verreibung bestehen aus 92 % Sorbit, 5 % Xylit und 3 % Mannit anstelle der Polyol-Zusammensetzung

Diese Kaugummi werden einer sensorischen Prüfung und einer penetrometrischen Messung unterzogen:
**A. Sensorische Prüfung der Kaumassen gem. angegebener Rezeptur nach dem Dreleck-Test (Triangle-test):**
- 1. Prüfung:: Zwei Proben mit dem Kaugummi gemäß Beispiel 10. Eine Probe mit Vergleichsbeispiel A
- 2. Prüfung:: Eine Probe gemäß Beispiel 10. Zwei Proben mit Vergleichsbeispiel B.
- Ergebnis:: Alle Prüfer stellten bei den Kauprüfungen ohne Fehler die Unterschiede fest und ordneten sämtliche Proben richtig zu.

Die demnach mit 100-prozentiger Trefferquote erkannten Unterschiede zeugen für eine hohe Signifikanz der Qualität der neuen Produkte.
Die Kaugummi mit Verwendung der Polyol-Kombinationen Beispiel 10 aus der Co-Sprühung wurden besonders bezüglich des Ankauverhaltens gut beschrieben. Weiterhin fielen sie durch nicht bröckeliges und nicht klebriges Verhalten auf. Die Verfestigung beim Auskauen verlief langsamer als bei den Vergleichen.
Zu dieser Prüfung wurden Proben herangezogen, die drei Wochen bei Raumtemperatur gelagert worden waren.
**B. Penetrometrische Messungen und allgemeine Beurteilungen der Kaugummi**
Beschreibung der Methode:
Mit dem Penetrometer wird die Textur von plastischen Stoffen gemessen. Es wird, je nach Festigkeit des zu prüfenden Produktes, ein kegelförmig zugespitzter Zylinder oder ein nadelartiger Splint in das Gerät einjustiert und mit einem definierten Gewicht beschwert.
Nach Auslösung des Meßvorganges wirkt der Eindringkörper durch die Schwerkraft eine eingestellte, genau gleichbleibende Zeitspanne lang auf das Versuchsprodukt ein.
Bei Kaumassen wird eine spezielle Nadel benutzt, die je nach Härte und Zähigkeit der Kaumassen mehr oder wenige tief in die 40 °C temperierte Masse eindringt.
Erwünscht ist eine Anfangsplastizität die, bei geringer Klebrigkeit problemlose Ausformung der Kaugummi ermöglicht. Erwünscht ist weiterhin eine ein nicht zu starkes Nachhärten der Fertigprodukte, das zu Bröckeln und schlechtem Ankauverhalten der Massen führen würde.
Ferner ist schon bei der Herstellung erwünscht, daß die Zusätze wie z.B. Zuckeraustauschstoffe leicht unterzukneten sind. Typische Symptome für Mängel bei der Einarbeitung sind deutlich hörbare Geräusche beim Einziehen und Entweichen der Luft in die Masse, das sog. "Schmatzen" oder "Knallen" während des Knetprozesses.

| | Eindringtiefe Penetrometer (mm) | | | Einarbeitung | Aussehen | Sensorik |
|---|---|---|---|---|---|---|
| | 1 Tag | 7 T. | 21 T. | (T. = Tage) | | |
| Beispiel 10 | 72 | 62 | 40 | kein Schmatzen, kein Knallen | glatt, biegsam | nicht klebend beim Ankauen; wenig Nachhärtung beim Auskauen |
| | | | | | | |
| Vergleichsbeispiel B | 81 | 54 | 33 | etwas Schmatzen und Knallen | fast glatt; wenig biegsam | etwas klebrig beim Ankauen; schnelle Nachhärtung beim Auskauen |
| | | | | | | |
| Vergleichsbeispiel A | 82 | 48 | 24 | deutliches Schmatzen und Knallen | etwas rauhe Oberfl.; nicht biegsam | rauh und klebrig beim Ankauen; schnelles Nachhärten beim Auskauen |

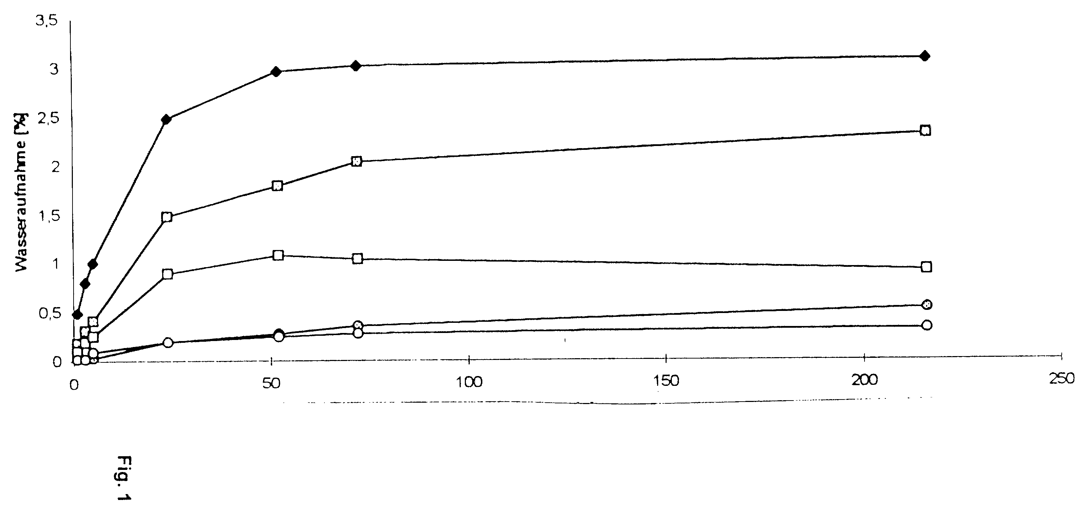

Die Komgröße der verwendeten Sorbitsorten war jeweils auf das übliche Spektrum für die Verwendung in Kaumassen eingestellt.
Die Unterschiede sind in jedem Fall. d.h. auch bei den penetrometrischen Messungen als signifikant zu bewerten.
In sämtlichen angegebenen Rezepturen kann auch mit Saccharin oder Aspartame aufgesüßte Polyolkombination oder auch eingefärbter Polyolkombination eingesetzt werden.
**C. Untersuchung der Hygroshopizität (Wasseraufnahme bei Lagerung im Hygrostaten)**
In einem Hygrostaten wird eine Probe der erfindungsgemäßen Zusammensetzung (hergestellt gemäß Beispiel 1) bzw. verschiedene Proben von unterschiedlich hergestelltem reinen Sorbit bzw. Sorbit-Verreibungen längere Zeit bei einer relativen Feuchtigkeit von 55 % aufbewahrt.
Die Wasseraufnahme der einzelnen Proben kann folgender Tabelle entnommen werden: 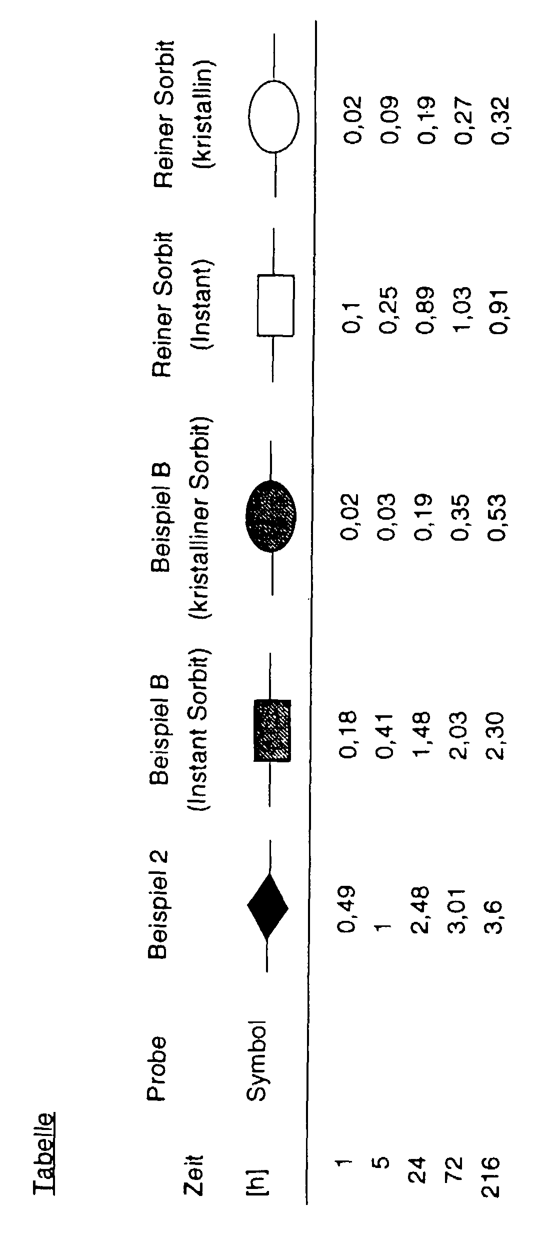
Fig. 1 zeigt der zeitlichen Verlauf der Wasseraufnahme der einzelnen Proben bei Lagerung im Hygrostaten.
Die für die einzelnen Proben verwendeten Symbole sind der oben angegebenen Tabelle zu entnehmen.
Die erfindungsgemäße Zusammensetzung nimmt wesentlich mehr Wasser auf als die anderen Proben, wodurch sie lange Zeit geschmeidig bleibt.

## Patentansprüche

1. Zusammensetzung im wesentlichen bestehend aus mindestens zwei Polyolen mit einem Mannit-Gehalt von weniger als 10 Gew.% erhältlich durch Co-Sprühtrocknung, dadurch gekennzeichnet, daß sie mindestens ein Hexit und mindestens ein Pentit enthält.

2. Zusammensetzung nach Anspruch 1, erhältlich durch Lösen von mindestens zwei Polyolen in Wasser und Versprühen des erhaltenen wässerigen Gemisches in einem Luftstrom mit einer Temperatur von 120 bis 300 °C.

3. Zusammensetzung nach Anspruch 1 oder 2, dadurch gekennzeichnet, daß Sorbit und Xylit oder Sorbit, Xylit und weitere Polyole, insbesondere Sorbit, Xylit und Mannit als Polyole eingesetzt werden.

4. Zusammensetzung nach Anspruch 3, dadurch gekennzeichnet, daß das Verhältnis von Sorbit zu Xylit in einem Bereich zwischen 50 : 50 bis 99 : 1, insbesondere zwischen 65 : 35 bis 98 : 2 liegt.

5. Zusammensetzung nach Anspruch 3, dadurch gekennzeichnet, daß das Verhältnis Sorbit : Xylit : Mannit in einem Bereich zwischen 90 : 1 : 9 bzw. 70 : 29 : 1 bis 98 : 1 : 1, insbesondere zwischen 90 : 2 : 8 bzw. 80 : 18 : 2 bis 94 : 1 : 5 bzw. 94 : 5 : 1, liegt.

6. Zusammensetzung nach einem der vorangehenden Ansprüche, dadurch gekennzeichnet, daß der Gehalt an Wasser niedriger als 1 Gew.-% liegt.

7. Zusammensetzung nach Anspruch 6, dadurch gekennzeichnet, daß sie bei Lagerung in einem Hygrostaten bei einer relativen Feuchte von 55 % innerhalb von 24 Stunden mehr als 2 % Wasser aufnimmt.

8. Komprimate, enthaltend eine Zusammensetzung nach einem der Ansprüche 1 bis 7.

9. Kaugummi, enthaltend eine Zusammensetzung nach einem der Ansprüche 1 bis 7.

10. Verfahren zur Herstellung einer im wesentlichen aus mindestens zwei Polyolen bestehenden Zusammenselzung, nach Anspruch 1, beinhaltend die folgenden Schritte:
a) Herstellen einer wäßrigen Lösung von mindestens zwei Polyolen, wobei die Lösung mindestens ein Hexit und mindestens ein Pentit, sowie weniger als 10 % Mannit bezogen auf den Gesamtpolyolgehalt enthält.
b) Versprühen der so erhaltenen Lösung in einem aufsteigendem Luftstrom mit einer Temperatur zwischen 120 und 300 °C, wobei das Wasser verdampft wird,
c) Isolierung der Zusammensetzung.

## Claims

1. Composition essentially consisting of at least two polyols having a mannitol content of less than 10 % by weight obtainable by co-spray-drying, characterized in that it comprises at least one hexitol and at least one pentitol.

2. Composition according to Claim 1, obtainable by dissolving at least two polyols in water and spraying the resulting aqueous mixture in an air stream having a temperature of 120 to 300°C.

3. Composition according to Claim 1 or 2, characterized in that sorbitol and xylitol, or sorbitol, xylitol and other polyols, in particular sorbitol, xylitol and mannitol, are used as polyols.

4. Composition according to Claim 3, characterized in that the ratio of sorbitol to xylitol is in a range between 50:50 and 99:1, in particular between 65:35 and 98:2.

5. Composition according to Claim 3, characterized in that the ratio of sorbitol:xylitol:mannitol is in a range between 90:1:9 or 70:29:1 and 98:1:1, in particular between 90:2:8 or 80:18:2 to 94:1:5 or 94:5:1.

6. Composition according to one of the preceding claims, characterized in that the water content is lower than 1 % by weight.

7. Composition according to Claim 6, characterized in that it absorbs more than 2 % water in the course of 24 hours during storage in a hygrostat at a relative humidity of 55 %.

8. Compacted articles comprising a composition according to one of Claims 1 to 7.

9. Chewing gum comprising a composition according to one of Claims 1 to 7.

10. Process for the preparation of a composition according to Claim 1 essentially consisting of at least two polyols, including the following steps:
a) preparing an aqueous solution of at least two polyols, where the solution comprises at least one hexitol and at least one pentitol and less than 10 % of mannitol, based on the total polyol content,
b) spraying the solution thus obtained in an ascending air stream at a temperature between 120 and 300°C, where the water is evaporated,
c) isolating the composition.

## Revendications

1. Composition constituée pour l'essentiel d'au moins deux polyols, ayant une teneur en mannitol inférieure à 10 % en poids, que l'on peut obtenir par séchage simultané par atomisation, caractérisée en ce qu'elle contient au moins un hexitol et au moins un pentitol.

2. Composition selon la revendication 1, que l'on peut obtenir par dissolution dans l'eau d'au moins deux polyols, et vaporisation du mélange aqueux ainsi obtenu, dans un courant d'air à une température de 120 à 300°C.

3. Composition selon la revendication 1 ou 2, caractérisée en ce qu'on utilise en tant que polyols du sorbitol et du xylitol ou du sorbitol, du xylitol et d'autres polyols, en particulier du sorbitol, du xylitol et du mannitol.

4. Composition selon la revendication 3, caractérisée en ce que le rapport du sorbitol au xylitol est compris dans l'intervalle de 20:50 à 99:1 et en particulier de 65:35 à 98:2.

5. Composition selon la revendication 3, caractérisée en ce que la proportion sorbitol:xylitol:mannitol est comprise dans l'intervalle de 90:1:9 ou 70:29:1 à 98:1:1, en particulier de 90:2:8, ou 80:18:2 à 94:1:5 ou 94:5:1.

6. Composition selon l'une des revendications précédentes, caractérisée en ce que la teneur en eau est inférieure à 1 % en poids.

7. Composition selon la revendication 6, caractérisée en ce que, lors d'un stockage dans un hygrostat en présence d'une humidité relative de 55 %, elle absorbe plus de % d'eau en 24 heures.

8. Comprimés contenant une composition selon l'une des revendications 1 à 7.

9. Gomme à mâcher contenant une composition selon l'une des revendications 1 à 7.

10. Procédé de préparation d'une composition selon la revendication 1, constituée pour l'essentiel d'au moins deux polyols, comprenant les étapes suivantes :
a) préparation d'une solution aqueuse d'au moins deux polyols, la solution contenant au moins un hexitol et au moins un pentitol, ainsi que moins de 10 % de mannitol par rapport à la quantité totale de polyol,
b) vaporisation de la solution ainsi obtenue dans un courant d'air ascendant à une température comprise entre 120 et 300°C, l'eau subissant une évaporation.
c) isolement de la composition.
